# EUROPEAN PATENT SPECIFICATION

(11) **EP 3 042 683 B1**
(45) Date of publication and mention of the grant of the patent: **30.08.2017**
(21) Application number: 14851791.5
(22) Date of filing: 17.09.2014
(51) Int. Cl.: A61M 16/20, A61M 16/00, A61M 16/06

(54) **OPENING AND CLOSING DEVICE AND BREATHING ASSISTANCE DEVICE**
ÖFFNUNGS- UND SCHLIESSVORRICHTUNG SOWIE BEATMUNGSVORRICHTUNG
DISPOSITIF D'OUVERTURE ET DE FERMETURE ET DISPOSITIF D'ASSISTANCE RESPIRATOIRE

(30) Priority: 11.10.2013 JP 2013213655
(43) Date of publication of application: 13.07.2016
(73) Proprietor: Metran Co., Ltd., Kawaguchi-shi, Saitama 332-0015 (JP)
(72) Inventor: NITTA Kazufuku, Kawaguchi-shi Saitama 332-0015 (JP)
(74) Representative: TBK
(86) International application number: PCT/JP2014/074569
(87) International publication number: WO 2015/053054

(56) References cited:
- WO-A1-2005/051468
- WO-A2-2013/040198
- JP-A- H02 125 179
- JP-A- 2007 092 507
- JP-A- 2013 090 823
- US-A- 5 067 487
- US-A1- 2006 266 361
- US-A1- 2010 326 447
- US-A1- 2012 161 045

## Description

### Technical Field

The present invention relates to an opening and closing device and a respiratory assistance device.

### Background Art

Respiratory assistance devices such as artificial respirators are used in medical practice. A typical respiratory assistance device includes an oxygen supply source such as an oxygen tank, an inspiratory pipe connected to the supply source, a mask attached to a tip of the inspiratory pipe, an expiratory pipe branched from the inspiratory pipe, an expiratory valve fixed to a tip of the expiratory pipe, etc. (for example, Japanese Patent Application Laid-Open Nos. Hei. 02-131765, Hei. 02-131773, Hei. 02-131774, and Hei. 05-245204).

Various methods such as a controlled ventilation (Controlled Ventilation) method used for a patient in the absence of spontaneous breathing (a patient under general anesthesia, during cardiopulmonary resuscitation, or in a critical condition) and an assisted ventilation (Assisted Ventilation) method in which a positive pressure (Positive Pressure) is created in an air passage in synchronization with the spontaneous breathing of a patient are employed for such respiratory assistance devices.

In a respiratory assistance device employing any of these methods, oxygen sent out from the oxygen tank is supplied to lungs as inspiratory air via the inspiratory pipe. The oxygen supplied to the lungs is then exhaled by the lungs as expiratory air. If the expiratory air is discharged into the expiratory pipe, a pressure in the expiratory pipe is increased. A control unit then receives a sensing signal from a pressure sensor having detected the pressure increase in the expiratory pipe and opens the expiratory valve. In this manner, the expiratory air is emitted to the outside from the expiratory pipe.

WO 2013/040198 A1 discloses a vent arrangement. In this vent arrangement, the opening and closing mechanism consists of two tubes inserted into each other, one being an inner vent member, the other being a cover member. In the opening and closing mechanism, an inner vent member is inserted into a cover member. The inner vent member comprises a vent portion, and the cover member comprises an opening. The inner vent member and the cover member take relative positions to cover or expose variable areas of the vent portion, where the relative positions are reached e.g. by manual manipulation or by piezo actuators.

US 2006/0266361 A1 discloses a ventilation interface. In the ventilation interface, an aperture is disposed on a lower wall of a cannula. A slide and a tab are positioned adjacent the aperture. The tab is moved in the slide vertically in order to increase or decrease the effective opening of the aperture by having walls to move and narrow the opening of the aperture.

WO 2005/051468 A1 discloses a vent system. A vent assembly is provided with two alternative vents. Selection between the first vent and the second vent is made by rotating or sliding a cover.

US 5 067 487 A discloses a manually adjustable air vent structure having a stopper. The stopper is in the form of a rotatable cap having an end wall and a skirt grippingly interfitting an annular lip. The lip surrounds a vent opening through a wall. The end wall has different sized vent openings, spaced about a rotation axis, and of selectively larger size to be selectively and individually brought into registration with the vent opening.

### Summary of Invention

### Technical Problem

A diaphragm valve has been known as an expiratory valve employed in such a respiratory assistance device. The diaphragm valve includes: a valve seat formed along a circumference of an opening of a hole through which the expiratory air passes (hereinafter referred to as an expiratory hole); and a valve element movable between a position supported by the valve seat and blocking the expiratory hole and a position away from the valve seat and opening the expiratory hole.

Although such a diaphragm valve is switchable between a state in which the entire expiratory hole is opened (hereinafter, referred to as an open state) and a state in which the entire expiratory hole is closed (hereinafter, referred to as a closed state), switching to a state in which part of the expiratory hole is opened, i.e., an intermediate state between the open state and the closed state is difficult to achieve.

The present invention has been made in view of the above problem, and it is an object of the present invention to provide an opening and closing device capable of switching among the open state, the closed state, and the intermediate state therebetween, and a respiratory assistance device including the opening and closing device.

### Solution to Problem

The object is solved by an opening and closing device comprising the features of claim 1. A respiratory assistance device comprising the opening and closing device is stated in claim 13. Further developments are subject-matter of the dependent claims.

Preferably, an aperture ratio of the flow hole when the opening and closing mechanism is located in the first position is smaller than that when the opening and closing mechanism is located in the second position, and a rate of change in an aperture length of the flow hole in the moving direction of the opening and closing mechanism is smaller at the first position than at the second position.

Preferably, a plurality of the flow holes are provided in the separating member. Moreover, the opening and closing mechanism preferably closes the plurality of the flow holes simultaneously.

Preferably, the opening and closing mechanism is movable among the first position, the second position, and a third position, the first position is disposed between the second position and the third position, the flow hole is closed at the first position, and the flow hole is opened at the second position and the third position. Moreover, a variation profile of the aperture length of the flow hole from the first position toward the third position is preferably different from a variation profile of the aperture length of the flow hole from the first position toward the second position. Alternatively, a variation profile of the aperture length of the flow hole from the first position toward the third position may be the same as a variation profile of the aperture length of the flow hole from the first position toward the second position.

A controller for controlling the deformation of the piezoelectric element is preferably included. Moreover, a part of the piezoelectric element serves as the opening and closing mechanism. Furthermore, a travel amount of the opening and closing mechanism preferably corresponds to a level of a signal input from the controller to the piezoelectric element.

An aperture area adjusting member movable along the separating surface is preferably further included, and a gap through which part of the flow hole is exposed is formed between the aperture area adjusting member and the opening and closing mechanism. Moreover, the aperture area adjusting member is preferably connected to the opening and closing mechanism.

A respiratory assistance device of the present invention includes the above-described opening and closing device, and the separating member is formed by a mask for covering a nose or a mouth, and a communicating pipe communicated with a space formed inside the mask in a worn state.

Preferably, the flow hole is formed in the mask. Alternatively, the flow hole may be formed in the communicating pipe. The flow hole preferably forms an expiratory pathway through which expiratory air exhaled from the nose or the mouth passes.

### Advantageous Effects of Invention

The opening and closing device of the present invention allows for switching among the open state, the closed state, and the intermediate state therebetween. Moreover, such an opening and closing device is suitable as an opening and closing device (for example, an expiratory valve) in a respiratory assistance device.

### Brief Description of Drawings

Fig. 1 is a schematic diagram illustrating a configuration of a respiratory assistance device according to a first embodiment of the present invention.
Fig. 2 includes perspective views illustrating the overview of an expiratory valve provided in a mask, where Fig. 2(A) shows a state in which the expiratory valve opens an expiratory hole, and Fig. 2(B) shows a state in which the expiratory valve blocks the expiratory hole.
Fig. 3 is a block diagram illustrating a hardware configuration of a control unit.
Fig. 4 is a block diagram illustrating a functional configuration of the control unit.
Fig. 5 is a graph showing a relationship between an input voltage V to a piezo element and a travel amount M of a tip of the expiratory valve, in which the horizontal axis represents the input voltage V to the piezo element, and the vertical axis represents the travel amount M of the tip of the expiratory valve.
Fig. 6(A) is an explanatory diagram illustrating the overview of the expiratory valve, the expiratory hole, and their surroundings when the input voltage V = V_{A}, Fig. 6(B) is an explanatory diagram illustrating the overview of the expiratory valve, the expiratory hole, and their surroundings when the input voltage V = V_{B}, and Fig. 6(C) is an explanatory diagram illustrating the overview of the expiratory valve, the expiratory hole, and their surroundings when the input voltage V = V_{C}.
Fig. 7 is an explanatory diagram showing a relationship between moving directions of the expiratory valve and the shape of the expiratory hole.
Fig. 8 is a graph showing a relationship between an input voltage V to the piezo element and an aperture ratio S/S_{MAX} of the expiratory hole in the case of Fig. 6, in which the horizontal axis represents the input voltage V to the piezo element, and the vertical axis represents the aperture ratio S/S_{MAX} of the expiratory hole.
Fig. 9 includes schematic diagrams illustrating exemplary control of the respiratory assistance device, where Fig. 9(A) shows a case where a user exhales air, and Fig. 9(B) shows a case where a user inhales air.
Fig. 10(A) is an explanatory diagram illustrating the overview of the expiratory valve, the expiratory hole, and their surroundings when the input voltage V = V_{A}, Fig. 10(B) is an explanatory diagram illustrating the overview of the expiratory valve, the expiratory hole, and their surroundings when the input voltage V = V_{B}, and Fig. 10(C) is an explanatory diagram illustrating the overview of the expiratory valve, the expiratory hole, and their surroundings when the input voltage V = V_{C}.
Fig. 11 is a graph showing a relationship between an input voltage V to the piezo element and an aperture ratio S/S_{MAX} of the expiratory hole in the case of Fig. 10, in which the horizontal axis represents the input voltage V to the piezo element, and the vertical axis represents the aperture ratio S/S_{MAX} of the expiratory hole.
Fig. 12 is an explanatory diagram illustrating the overview of the expiratory valve, the expiratory holes, and their surroundings.
Fig. 13 is a graph showing a relationship between an input voltage V to the piezo element and an aperture ratio S/S_{MAX} of the expiratory hole in the case of Fig. 12, in which the horizontal axis represents the input voltage V to the piezo element, and the vertical axis represents the aperture ratio S/S_{MAX} of the expiratory hole.
Fig. 14 is an explanatory diagram illustrating the overview of the expiratory valve, the expiratory holes, and their surroundings.
Fig. 15 is a graph showing a relationship between an input voltage V to the piezo element and an aperture ratio S/S_{MAX} of the expiratory hole in the case of Fig. 14, in which the horizontal axis represents the input voltage V to the piezo element, and the vertical axis represents the aperture ratio S/S_{MAX} of the expiratory hole.
Fig. 16(A) is an explanatory diagram illustrating the overview of the expiratory valve, the expiratory hole, and their surroundings when the input voltage V = V_{A}, Fig. 16(B) is an explanatory diagram illustrating the overview of the expiratory valve, the expiratory hole, and their surroundings when the input voltage V = V_{B}, and Fig. 16(C) is an explanatory diagram illustrating the overview of the expiratory valve, the expiratory hole, and their surroundings when the input voltage V = Vc.

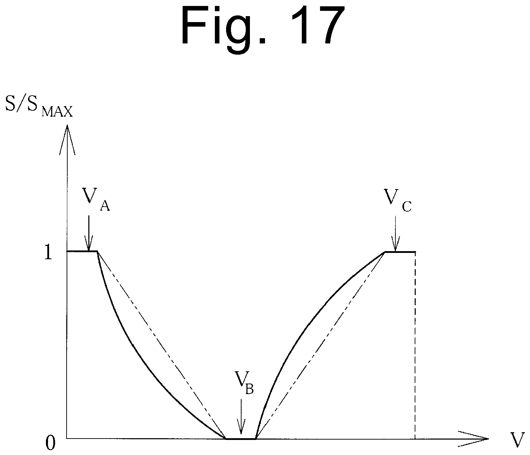

Fig. 17 is a graph showing a relationship between an input voltage V to the piezo element and an aperture ratio S/S_{MAX} of the expiratory hole in the case of Fig. 16, in which the horizontal axis represents the input voltage V to the piezo element, and the vertical axis represents the aperture ratio S/S_{MAX} of the expiratory hole.
Fig. 18(A) is an explanatory diagram illustrating the overview of an expiratory valve in an opened state, and Fig. 18(B) is an explanatory diagram illustrating the overview of the expiratory valve in a blocked state.
Fig. 19(A) is an explanatory diagram illustrating a state in which only an aperture area adjusting member blocks part of the expiratory hole, and Fig. 19(B) is an explanatory diagram illustrating a state in which each of an opening and closing mechanism and the aperture area adjusting member blocks part of the expiratory hole.
Fig. 20 is a graph showing a relationship between an input voltage V to the piezo element and an aperture ratio S/S_{MAX} of the expiratory hole in the case of Fig. 19, in which the horizontal axis represents the input voltage V to the piezo element, and the vertical axis represents the aperture ratio S/S_{MAX} of the expiratory hole.
Fig. 21(A) is an explanatory diagram illustrating the overview of an expiratory valve in an opened state, and Fig. 21(B) is an explanatory diagram illustrating the overview of the expiratory valve in a blocked state.
Fig. 22 is a schematic diagram illustrating a configuration of a respiratory assistance device according to the second embodiment of the present invention.
Fig. 23(A) is a cross-sectional view illustrating an exemplary configuration of a micro pump, and Fig. 23(B) is a graph showing pressure-flow rate lines of the micro pump. Description of Embodiments

Embodiments of the present invention will now be described below with reference to the accompanying drawings.

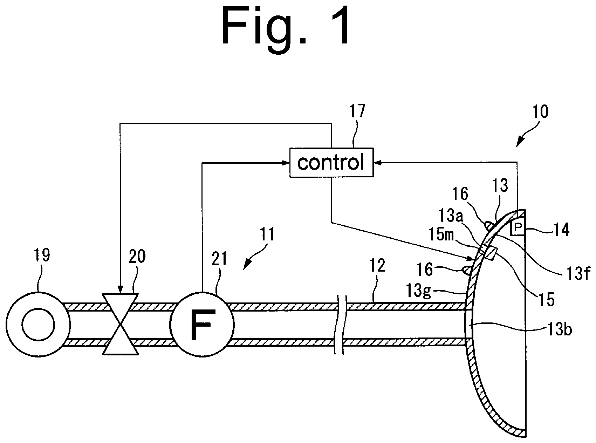

Fig. 1 illustrates an exemplary configuration of a respiratory assistance device 10 for medical use according to the first embodiment of the present invention. The respiratory assistance device 10 includes: a mask 13 having an expiratory hole 13a and an inspiratory hole 13b; an inspiratory pipe 12 inserted into the inspiratory hole 13b; a supply source 11 provided in the inspiratory pipe 12 and sending out an inspiratory gas; an air gage 14 for measuring a gas pressure in the mask 13; an expiratory valve 15 provided in the mask 13 and serving as an opening and closing mechanism for the expiratory hole 13a; a plurality of safety members 16 provided around the expiratory hole 13a so as to protrude toward the outer side of an expiratory pathway; and a control unit 17 for performing overall control on the entire device. The mask 13 and the expiratory valve 15 together form an opening and closing device.

The mask 13 is a wearable device that covers a mouth and a nose and serves as a member for separating a mouth and a nose from external space (a separating member). The inspiratory pipe 12 and the mask 13 are communicated with each other via the inspiratory hole 13b. An inspiratory pathway is formed by the inspiratory pipe 12, the inspiratory hole 13b, and the mask 13. The expiratory pathway is formed by the mask 13 and the expiratory hole 13a. Note that the mask 13 may be a wearable device that covers either a mouth or a nose.

The supply source 11 includes: a gas tank 19 that retains a gas such as air or oxygen in a compressed state; a regulating valve 20 for regulating a flow rate of the gas sent out from the gas tank 19; and a flowmeter 21 for measuring the flow rate of the gas regulated by the regulating valve 20. The regulating valve 20 is controlled on the basis of sensing data (measured results, sensing signals) of the air gauge 14 and the flowmeter 21. The regulating valve 20 is not limited to any particular type of valve, and may be an electric valve, an electromagnetic valve having a high response speed, or the like. The flowmeter 21 outputs the sensing data to the control unit 17.

The inspiratory pipe 12 is formed by a bellows tube made of a resin. The inspiratory pipe 12 forms a space together with the mask 13 worn by a patient to serve as a pathway for the gas sent out from the supply source 11. A gas pressure inside the inspiratory pipe 12 coincides with a gas pressure in the mask 13 worn by the patient in a steady state. The air gauge 14 outputs the sensing data to the control unit 17.

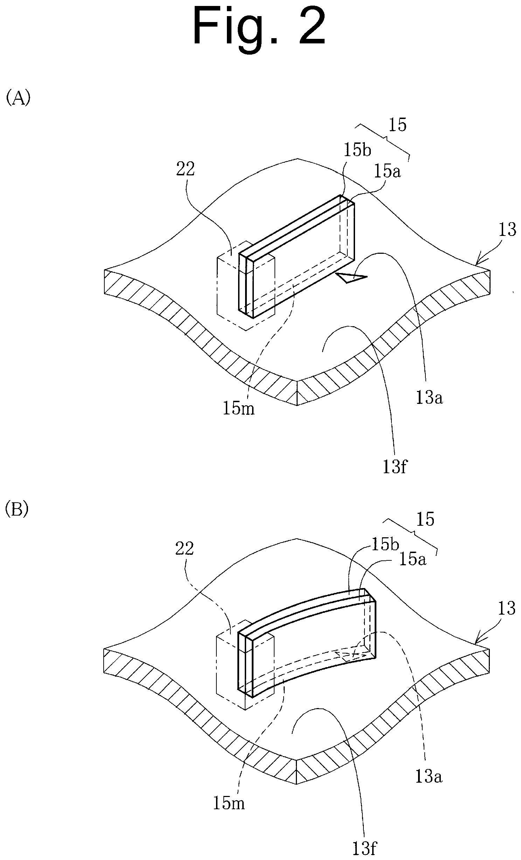

As shown in Fig. 2, the expiratory valve 15 emits the gas in the mask 13 to the outside of the mask 13 by opening and closing the expiratory hole 13a in the form of a slit and functions as a check valve for preventing a back-flow of such a released gas. The plate-shaped expiratory valve 15 is a valve having a monomorph (unimorph) structure in which a piezo element (piezoelectric element) 15a, which is displaced according to an amount of applied voltage, is layered on a metal plate 15b and having a one-end supported (cantilever) structure. Furthermore, the respiratory assistance device 10 has a fixing member 22 for fixing one end of the expiratory valve 15 to the mask 13. The fixing member 22 is provided so as to erect from an inner surface 13f of the mask 13. The one end of the expiratory valve 15 is fixed to the mask 13 by the fixing member 22 with a position erecting from the inner surface 13f. A cantilever length of the expiratory valve 15 is preferably about 30 mm or more and about 40 mm or less. A stroke by which the expiratory valve 15 is displaced is preferably 2 mm or more and 3 mm or less. Note that the piezo element may have a both-end supported structure.

The piezo element 15a is deformable between an extended state (see Fig. 2(A)) and an arched state (see Fig. 2(B)) according to the level of an input voltage. When the piezo element 15a is in the arched state, a side surface 15m of the expiratory valve 15 is located on an aperture plane of the expiratory hole 13a, thus blocking the expiratory hole 13a. When the piezo element 15a is in the extended state, on the other hand, the side surface 15m of the expiratory valve 15 is away from the expiratory hole 13a, thus opening the expiratory hole 13a (see Fig. 2(A)). In this manner, the expiratory valve 15 is switchable between the state in which the expiratory hole 13a is opened and the state in which the expiratory hole 13a is blocked, by the deformation of the piezo element 15a along the side surface 15m. In this manner, the expiratory valve 15 can transition, due to the deformation of the piezo element 15a, between the state in which the expiratory hole 13a formed in the mask 13 is opened (hereinafter, referred to as a opened state) (see Fig. 2(A)) and the state in which the expiratory hole 13a is blocked by the side surface 15m of the expiratory valve 15 (hereinafter, referred to as a blocked state) (see Fig. 2(B)). Note that the side surface 15m of the expiratory valve 15 may slide on the inner surface 13f by the deformation of the piezo element 15a along the side surface 15m. Additionally, the inner surface 13f may be a flat surface or a curved surface.

As will be described later, the piezo element 15a is in the arched state under the application of a voltage and in the extended state without the application of a voltage. Note that the piezo element 15a may be configured to be in the extended state under the application of a voltage and in the arched state without the application of a voltage. Although the expiratory valve 15 having the monomorph structure has been discussed here, it is apparent that a bimorph structure including two piezo elements attached to each other may be employed instead.

Referring back to Fig. 1, if the expiratory hole 13a is covered by an object outside the mask 13, the actuation of the expiratory valve 15 fails to secure the expiratory pathway. In view of this, it is preferable that the mask 13 be provided with the safety members 16. The safety members 16 are formed so as to protrude from an outer surface 13g of the mask 13 and arranged to be dotted near the expiratory hole 13a. This can form a gap between the aperture plane of the expiratory hole 13a on the outer surface 13g side and the object covering the expiratory hole 13a. Thus, the expiratory pathway can be secured by the actuation of the expiratory valve 15.

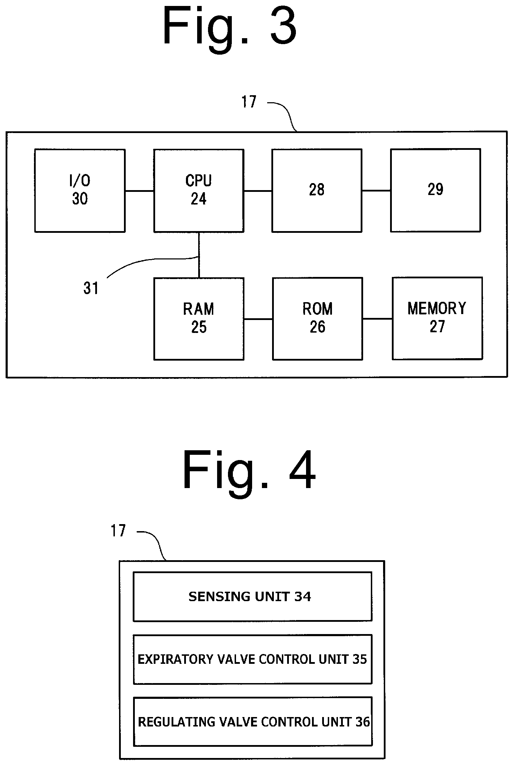

As shown in Fig. 3, the control unit 17 includes a CPU 24, a first storage medium 25, a second storage medium 26, a third storage medium 27, an input device 28, a display device 29, an input and output interface 30, and a bus 31.

The CPU 24 is what is called a central processing unit. Various programs are executed by the CPU 24 to implement various functions of the control unit 17. The first storage medium 25 is what is called a RAM (Random Access Memory) and used as a work area of the CPU 24. The second storage medium 26 is what is called a ROM (Read Only Memory) and stores a basic operating system executed by the CPU 24. The third storage medium 27 is configured, for example, by a hard disk device with a built-in magnetic disk, a disk device for accommodating a CD, a DVD, or a BD, and a non-volatile semiconductor flash memory device. The third storage medium 27 stores various programs to be executed by the CPU 24.

The input device 28, which is an input key, a keyboard, or a mouse, inputs a variety of information. The display device 29, which is a display, displays various operational states. A power supply and control signals for operating the expiratory valve 15 are input to and output from the input and output interface 30. The input and output interface 30 further acquires data, such as a program, from an external personal computer. The bus 31 serves as wiring for integrally connecting, for example, the CPU 24, the first storage medium 25, the second storage medium 26, the third storage medium 27, the input device 28, the display device 29, and the input and output interface 30, and the like to perform communication among them.

Fig. 4 shows a functional configuration obtained by executing a control program stored in the control unit 17 by the CPU 24. The control unit 17 includes, as a functional configuration, a sensing unit 34, an expiratory valve control unit 35, and a regulating valve control unit 36. The sensing unit 34 constantly acquires, and then transmits to the expiratory valve control unit 35, the sensing data of the air gauge 14. Additionally, the sensing unit 34 constantly acquires, and then transmits to the regulating valve control unit 36, the sensing data of the air gauge 14 and the flowmeter 21. The expiratory valve control unit 35 refers to the sensing data of the air gauge 14 and outputs a control signal based on this sensing data to the piezo element 15a. The regulating valve control unit 36 refers to the sensing data of the air gauge 14 and the flowmeter 21, and outputs a control signal based on this sensing data to the regulating valve 20. In this manner, a predetermined flow rate value can be obtained.

The details of the expiratory valve 15 and the expiratory hole 13a will be described next.

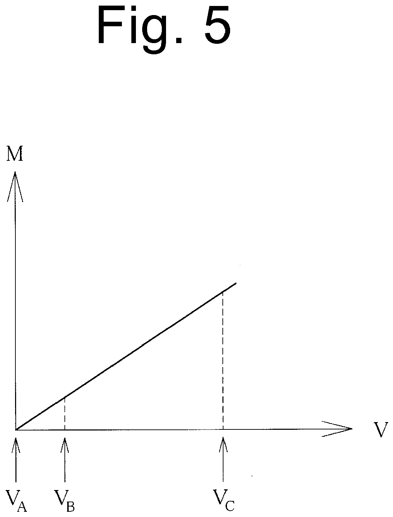

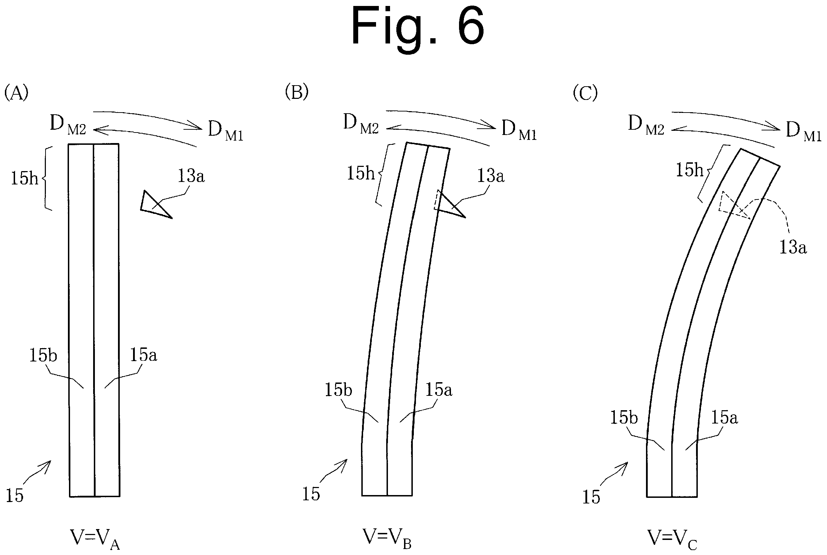

As shown in Fig. 5, a travel amount M of a tip 15h (see Fig. 6) of the expiratory valve 15 is directly proportional to the level of a control signal output from the expiratory valve control unit 35, i.e., the magnitude of an input voltage V. Therefore, the tip 15h of the expiratory valve 15 moves in a direction D_{M1} as the input voltage V increases, and moves in a direction D_{M2} as the input voltage V decreases.

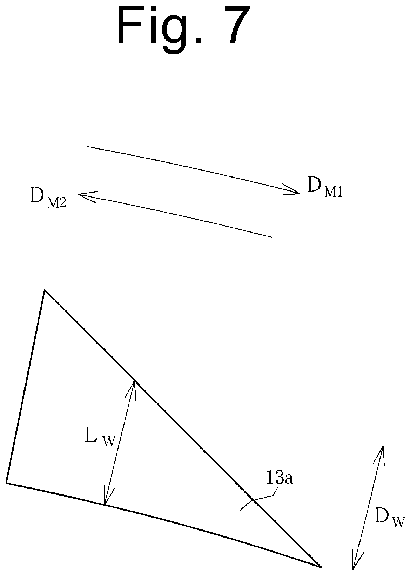

If the direction perpendicular to the direction D_{M1} is defined as a width direction D_{W}, a length L_{W} of the expiratory hole 13a in the width direction D_{W} decreases toward the direction D_{M1} (see Fig. 7) .

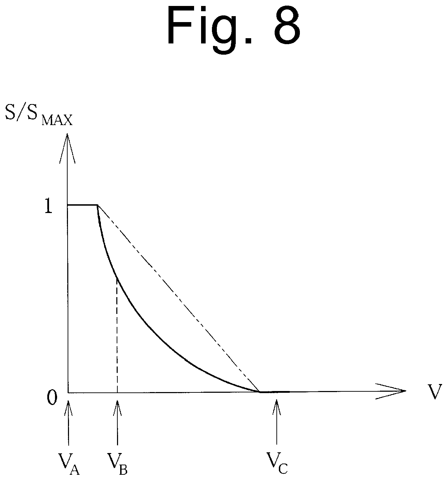

Here, if the aperture area of the expiratory hole 13a itself is denoted by S_{MAX}, an area of the expiratory hole 13a not covered by the expiratory valve 15 (opening area) is denoted by S, and an aperture ratio of the expiratory hole 13a is denoted by S/S_{MAX}, the aperture ratio of the expiratory hole 13a decreases non-linearly as the input voltage V increases (see Fig. 8). In other words, the gradient of the graph in Fig. 8 is in a negative value range and increases as the input voltage V increases.

A control example for the respiratory assistance device 10 will be described next.

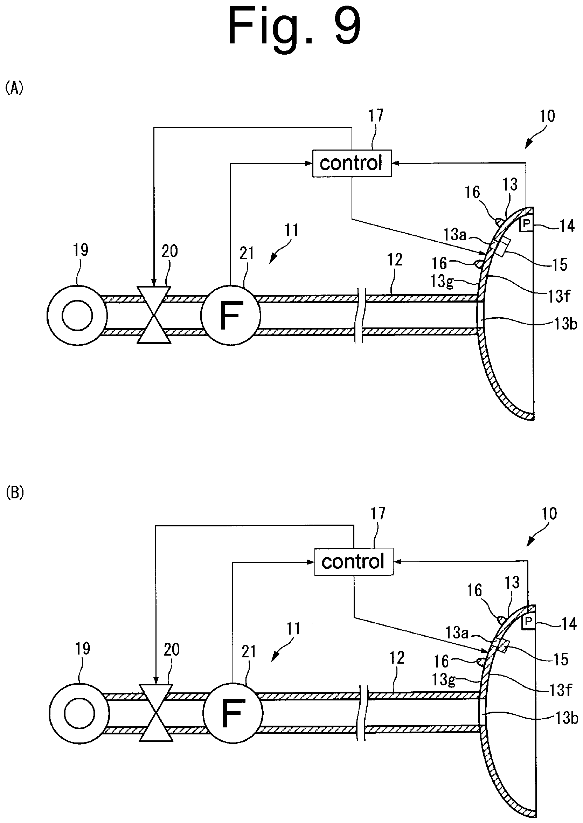

First, if expiratory air is exhaled from a mouth or nose wearing the mask 13, the pressure inside the mask 13 increases. If the pressure inside the mask 13 is increased, the increased value is sensed by the air gauge 14. The sensing data is output to the control unit 17. On the basis of the sensing data, the control unit 17 controls the expiratory valve 15. More specifically, the control unit 17 operates the expiratory valve 15 to open the expiratory hole 13a as shown in Fig. 9(A). The expiratory air is released to the outside of the mask 13 through the expiratory hole 13a.

The release of the expiratory air to the outside of the mask 13 causes the pressure inside the mask 13 to decrease. If the pressure inside the mask 13 is decreased, the decreased value is sensed by the air gauge 14. The sensing data is output to the control unit 17. On the basis of the sensing data, the control unit 17 controls the expiratory valve 15. More specifically, the control unit 17 operates the expiratory valve 15 to block the expiratory hole 13a. This forms an enclosed space inside the mask 13, thus enabling an inspiratory operation.

Subsequently, if inspiration is performed by the mouth or nose wearing the mask 13, the pressure inside the mask 13 is decreased. If the pressure inside the mask 13 is decreased, the decreased value is sensed by the air gauge 14. The sensing data is output to the control unit 17. On the basis of the sensing data, the control unit 17 controls the supply source 11. More specifically, the control unit 17 opens the regulating valve 20 to send out the gas from the gas tank 19 as the inspiratory air as shown in Fig. 9(B). Thereafter, the pressure inside the mask 13 is increased. If the pressure inside the mask 13 is increased, the increased value is sensed by the air gauge 14. The sensing data is output to the control unit 17. On the basis of the sensing data, the control unit 17 controls the supply source 11. More specifically, the control unit 17 closes the regulating valve 20 to stop the sending out of the gas from the gas tank 19 as the inspiratory air. Thereafter, the expiratory operation and the inspiratory operation are repeated in the same manner.

Here, if the deformation directions of the piezo element 15a correspond to the direction away from the inner surface 13f and the direction closer to the inner surface 13f, such deformation directions are substantially parallel to the direction of a force generated by a pressure difference between the inside and the outside of the mask 13. Thus, the piezo element 15a is easily deformed by such a force generated by a pressure difference between the inside and the outside of the mask 13. In the respiratory assistance device 10 described above, on the other hand, the expiratory valve 15 is disposed so that the deformation directions of the piezo element 15a correspond to directions along the inner surface 13f. Thus, the deformation directions of the piezo element 15a are substantially perpendicular to the direction of the force generated by the pressure difference between the inside and the outside of the mask 13. Consequently, the piezo element 15a is hardly deformed by such a force generated by the pressure difference between the inside and the outside of the mask 13. In this manner, the expiratory valve 15 has rigidity enough to resist the pressure from the expiratory hole 13a. Moreover, since the piezo element is simply employed as the expiratory valve 15 itself, an increase in procurement cost or processing cost can be avoided. Furthermore, the expiratory valve 15 having such a structure is switchable among an intermediate state in which part of the expiratory hole 13a is closed as well as the opened state and the blocked state.

The length L_{W} of the expiratory hole 13a in the width direction D_{W} decreases toward the direction D_{M1} as shown in Fig. 7. Thus, the aperture ratio of the expiratory hole 13a can be fine-tuned more easily in a range where the expiratory hole 13a has a small aperture ratio than in a range where the expiratory hole 13a has a large aperture ratio.

Moreover, the expiratory valve 15 is disposed so that the deformation directions of the piezo element 15a correspond to the directions along the inner surface 13f as shown in Fig. 2. Thus, the fully-opened state of the expiratory hole 13a can be easily obtained with a smaller deformation amount of the piezo element 15a as compared with the case where the deformation directions of the piezo element 15a correspond to the direction away from the inner surface 13f and the direction closer to the inner surface 13f.

Furthermore, since the expiratory valve 15 is configured to include the piezo element 15a, such an expiratory valve 15 has longer endurance, and is thus less likely to break, as compared with a case where an electromagnetic valve is employed as the expiratory valve.

Thus, the application of the present invention allows a patient with, for example, sleep apnea syndrome to use such a device as a home artificial respirator.

Moreover, the expiratory valve 15 is in the state in which the expiratory hole 13a is opened under no application of a voltage to the piezo element 15a. Thus, even when the expiratory valve 15 fails to operate due to breakdown or the like, such an expiratory valve 15 is put in the state in which the expiratory hole 13a is opened, thus securing the expiratory pathway.

Moreover, since the expiratory valve 15 is provided in the mask 13, the expiratory valve 15 can respond to the expiratory operation quickly, thus reducing a burden on the patient.

Furthermore, since the expiratory valve 15 is provided inside the mask 13, interference between the expiratory valve 15 and an object outside the mask 13 can be prevented from occurring. Note that the expiratory valve 15 may be provided on the outer surface of the mask 13.

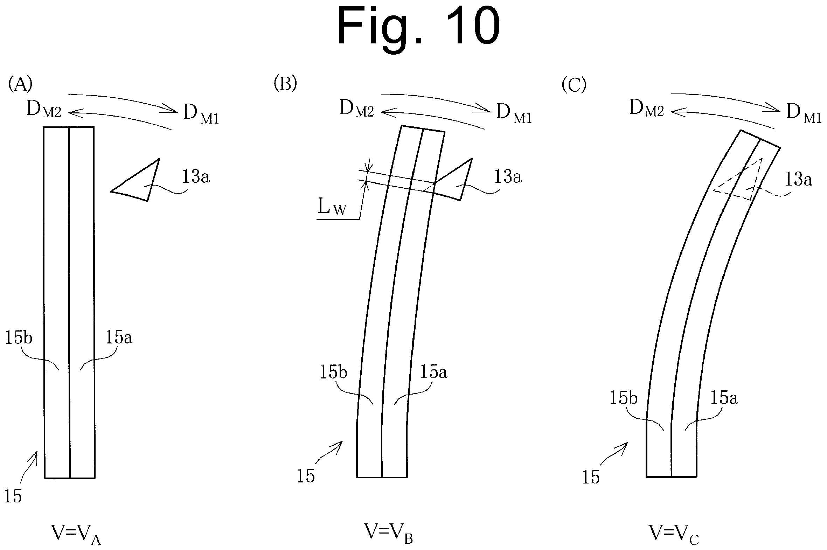

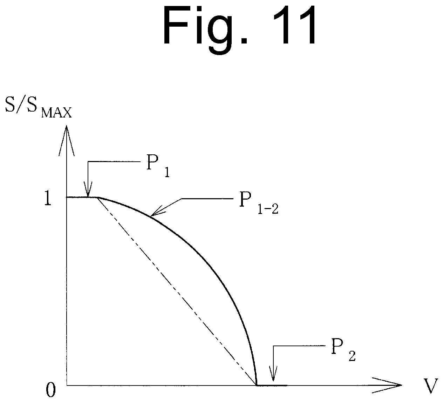

Although the length L_{W} of the expiratory hole 13a in the width direction D_{W} decreases toward the direction D_{M1} as shown in Fig. 7 in the above-described embodiment, the present invention is not limited thereto. For example, the length L_{W} of the expiratory hole 13a in the width direction D_{W} may increase toward the direction D_{M1} as shown in Fig. 10. In this case, the gradient of a graph in Fig. 11 is in a negative value range and decreases as the input voltage V increases.

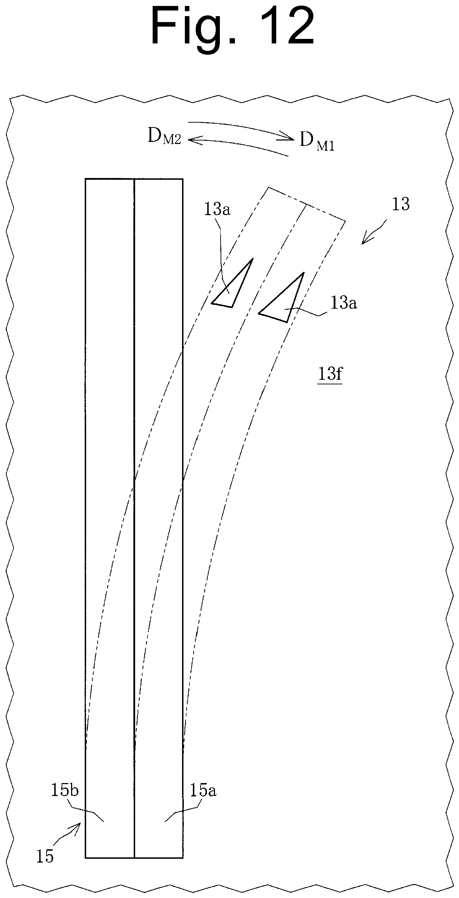

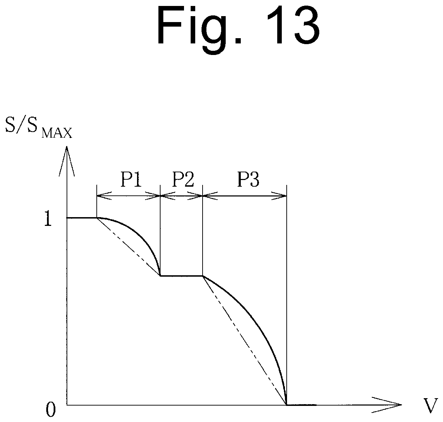

Alternatively, a plurality of expiratory holes 13a with a predetermined gap therebetween in the direction D_{M1} or the direction D_{M2} may be provided in the mask 13 as shown in Fig. 12. In this case, the expiratory valve 15 is switchable among a state in which all of the plurality of expiratory holes 13a are opened, a state in which part of the plurality of expiratory holes 13a is closed and the remaining part thereof is opened, and a state in which all of the plurality of expiratory holes 13a are closed. The length L_{W} of each of the plurality of expiratory holes 13a shown in Fig. 12 increases toward the direction D_{M1}. In such a case, a graph (see Fig. 13) showing the relationship between the input voltage V and the aperture ratio of the expiratory hole 13a includes: two parts P1 and P3 over each of which the gradient is in a negative value range and decreases as the input voltage V increases; and a part P2 over which the gradient is 0. The part P2 is disposed between the part P1 and the part P3. The range of the input voltage V having the part P2 can be adjusted by the gap between the plurality of expiratory holes 13a.

Although the lengths L_{W} of the plurality of expiratory holes 13a increase toward the direction D_{M1} in Fig. 12, the lengths L_{W} of the plurality of expiratory holes 13a may decrease toward the direction D_{M1} in the present invention.

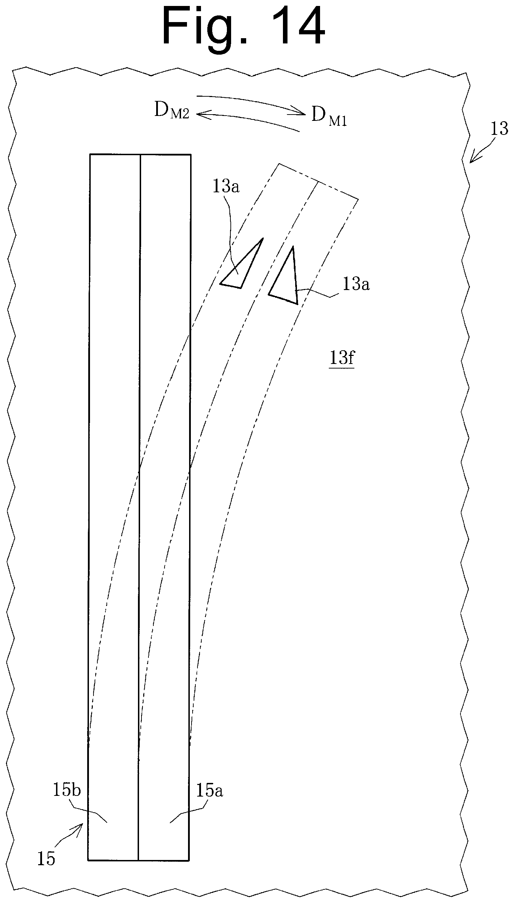

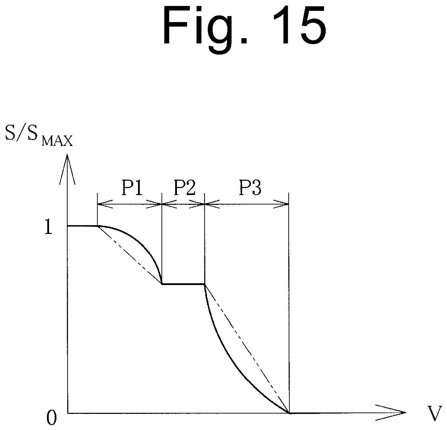

Alternatively, the length L_{W} of one of the plurality of expiratory holes 13a (the expiratory hole positioned upstream in the direction D_{M1}) may increase toward the direction D_{M1}, whereas the length L_{W} of the other one (the expiratory hole positioned downstream in the direction D_{M1}) may decrease toward the direction D_{M1} (see Fig. 14). In this case, a graph (see Fig. 15) showing a relationship between the input voltage V and the aperture ratio of the expiratory hole 13a includes: a part P1 over which the gradient is in a negative value range and decreases as the input voltage V increases; a part P3 over which the gradient is in a negative value range and increases as the input voltage V increases; and a part P2 disposed between the part P1 and the part P3, over which the gradient is 0.

The accuracy of control for aperture ratios can be set individually for each predetermined voltage range by setting the shape of the expiratory hole 13a so that the length L_{W} thereof increases or decreases toward the direction D_{M1} as described above.

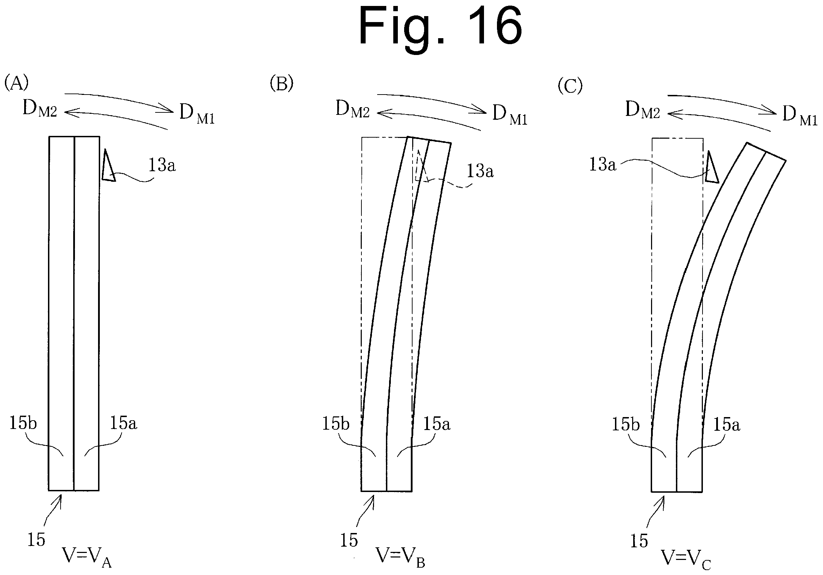

In the above-described embodiment, the position of the tip 15h to cause the opened state and the position of the tip 15h to cause the blocked state are arranged in this order toward the direction D_{M1}. However, the present invention is not limited thereto. For example, a first opening position of the tip 15h to cause the opened state of the expiratory hole 13a (V = V_{A}, see Fig. 16(A)), a blocking position of the tip 15h to cause the blocked state of the expiratory hole 13a (V = V_{B}, see Fig. 16(B)), and a second opening position of the tip 15h to cause the opened state of the expiratory hole 13a (V = V_{C}, see Fig. 16(C)) may be arranged in this order toward the direction D_{M1}. Moreover, the expiratory hole 13a is shaped so that the length L_{W} thereof in the width direction D_{W} decreases toward the direction D_{M1} and increases toward the direction D_{M2}. Thus, the profile of change in aperture ratio when the tip 15h is moved from the blocking position (see Fig. 16(B)) to the first opening position (see Fig. 16(A)) is different from that when the tip 15h is moved from the blocking position to the second opening position (see Fig. 16(C)). In other words, this can yield different profiles for change in aperture ratio. Furthermore, the blocking position is set between the first opening position and the second opening position. Thus, when the tip 15h is at the blocking position, one of the profiles for change in aperture ratio can be selected by the input value of the input voltage V.

In the above-described embodiment, the expiratory hole 13a is shaped so as to be symmetric about an axis extending in the width direction D_{w}. Consequently, the different profiles for change in aperture ratio can be obtained. However, the present invention is not limited thereto. The shape of the expiratory hole 13a may be symmetric about the axis extending in the width direction D_{W}. In such a case, two identical profiles for change in aperture ratio can be obtained.

Although the opening and closing device in the above-described embodiment employs the tip 15h of the deformable expiratory valve 15 as an opening and closing mechanism, the present invention is not limited thereto. A valve provided to the tip 15h of the deformable expiratory valve 15 may be used as an opening and closing mechanism. Examples of use in such a case include the following. The opening and closing of the expiratory hole 13a during a normal operation are performed on the lower-voltage side, i.e., between the first opening position and the blocking position. Under an abnormal condition, i.e., when a high voltage is input due to some trouble, it is possible to open the expiratory hole 13a. This eliminates the risk of blocking the expiratory hole 13a even when a high voltage is input due to some trouble.

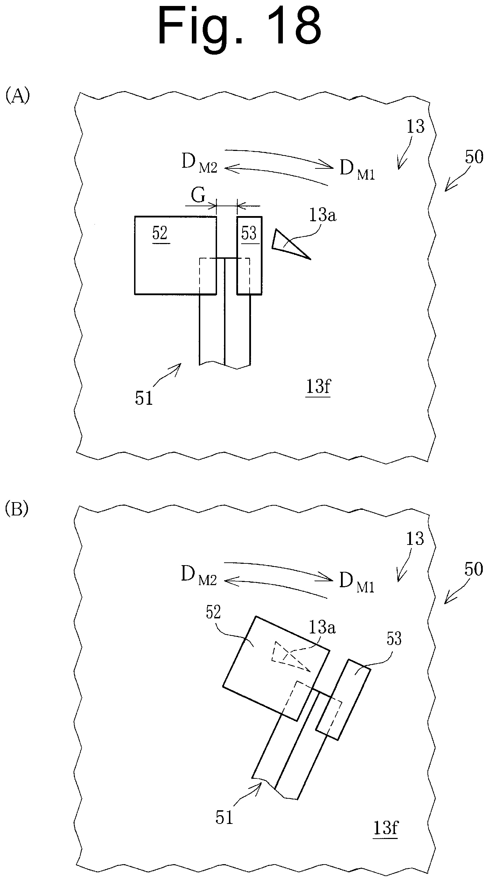

Note that the opening and closing device of the present invention may be an opening and closing device 50 as shown in Fig. 18. The opening and closing device 50 includes the mask 13, a deformable member 51, an opening and closing mechanism 52 capable of opening and closing the expiratory hole 13a, and an aperture area adjusting member 53 capable of adjusting the aperture area of the expiratory hole 13a.

The deformable member 51 corresponds to the expiratory valve 15 shown in Fig. 2, for example. Referring back to Fig. 18, the opening and closing mechanism 52 is provided to the deformable member 51. Thus, the opening and closing mechanism 52 can move over the expiratory hole 13a along the inner surface 13f. Furthermore, the surface of the opening and closing mechanism 52 on the inner surface 13f side has a shape and a size capable of covering the aperture plane of the expiratory hole 13a. Thus, the opening and closing mechanism 52 is movable between a state in which the expiratory hole 13a is opened (see Fig. 18(A)) and a state in which the expiratory hole 13a is closed (see Fig. 18(B)).

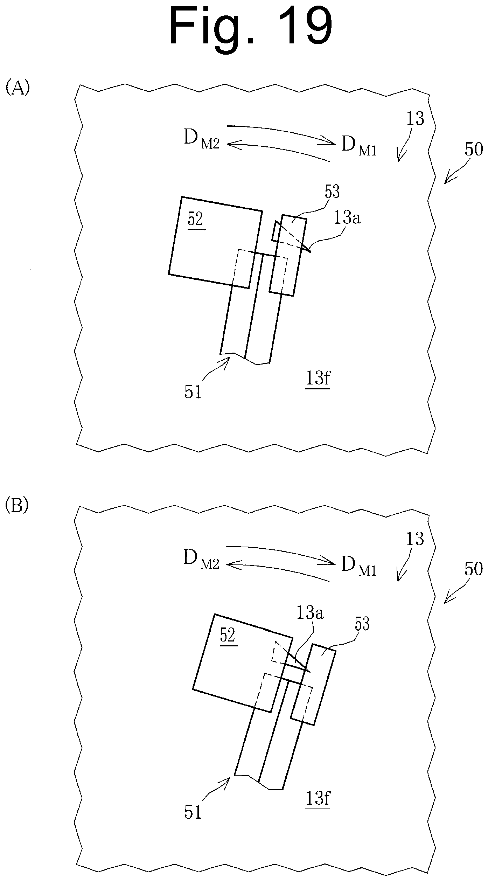

The aperture area adjusting member 53 is also movable over the expiratory hole 13a along the inner surface 13f because it is provided to the deformable member 51. Moreover, the aperture area adjusting member 53 is apart from the opening and closing mechanism 52 by a predetermined gap G. The gap G preferably has a size just enough to cause the exposure of not all but part of the expiratory hole 13a (see Fig. 19).

The function of the opening and closing device 50 will be described next.

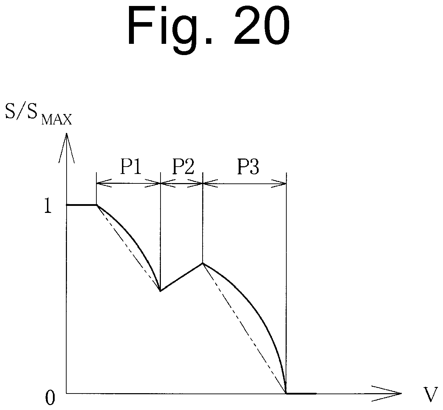

As shown in Figs. 18 and 19, as the input voltage V from the expiratory valve control unit 35 increases, the opening and closing device 50 sequentially switches among a state in which the entire aperture plane of the expiratory hole 13a is opened (see Fig. 18(A)), a state in which only the aperture area adjusting member 53 blocks part of the expiratory hole 13a (see Fig. 19(A)), a state in which each of the opening and closing mechanism 52 and the aperture area adjusting member 53 blocks part of the expiratory hole 13a (see Fig. 19(B)), and a state in which the entire aperture plane of the expiratory hole 13a is blocked (see Fig. 18(B)). As the input voltage V from the expiratory valve control unit 35 decreases, the opening and closing device 50 sequentially switches in the reverse order. Consequently, a graph (see Fig. 20) showing a relationship between the aperture ratio of the expiratory hole 13a and the input voltage V includes: parts P1 and P3 each exhibiting a decreasing function; and a part P2 exhibiting an increasing function. The profile for change in aperture ratio can be appropriately adjusted by the opening and closing mechanism 52, the shape of the aperture area adjusting member 53, the shape of the expiratory hole 13a, and the gap G.

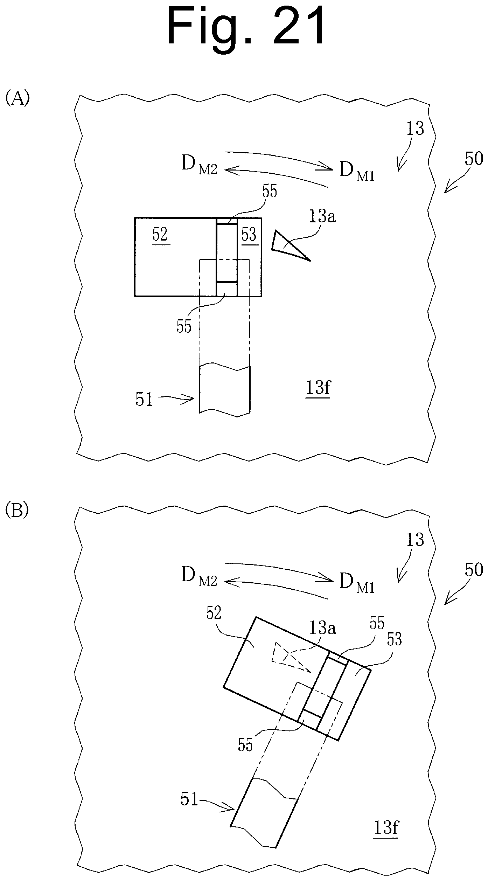

Although the opening and closing mechanism 52 and the aperture area adjusting member 53 are spaced apart from each other in the above-described embodiment, the present invention is not limited thereto. A connecting member 55 may be used to directly connect the opening and closing mechanism 52 and the aperture area adjusting member 53 (see Fig. 21). Furthermore, the opening and closing mechanism 52 and the aperture area adjusting member 53, or the opening and closing mechanism 52, the aperture area adjusting member 53, and the connecting member 55 may be integrally formed.

Although the single deformable member 51 is used to move the opening and closing mechanism 52 and the aperture area adjusting member 53 in the above-described embodiment, the present invention is not limited thereto. One deformable member 51 may be used to move the opening and closing mechanism 52, and another deformable member 51 may be used to move the aperture area adjusting member 53.

Note that two expiratory valves 15 may be employed. One tip 15h thereof (see Fig. 6) may be used as an opening and closing mechanism and the other tip 15h thereof (see Fig. 6) may be used as an aperture area adjusting member.

Although the length L_{W} of the single expiratory hole 13a in the width direction D_{W} is defined as an "aperture length" in the above-described embodiment, the present invention is not limited thereto. When a plurality of expiratory holes 13a are provided in the mask 13, the sum of the lengths L_{W} of the expiratory holes 13a in the width direction D_{w} may be defined as an "aperture length."

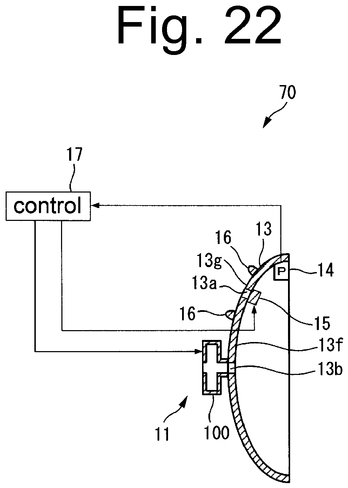

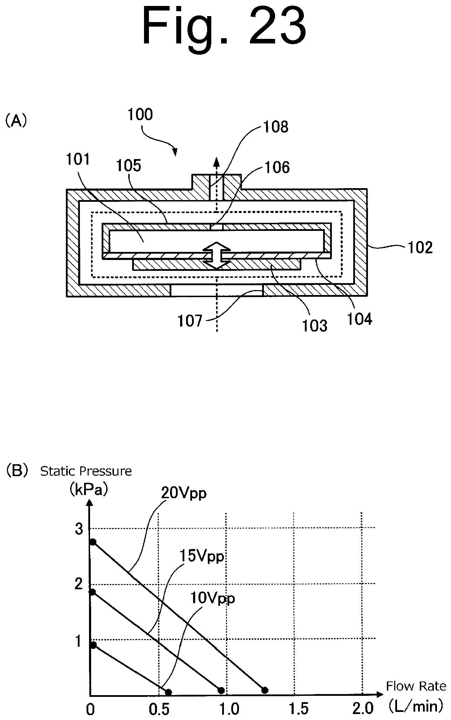

Fig. 22 illustrates an exemplary configuration of a respiratory assistance device 70 according to a second embodiment. The respiratory assistance device 70 includes a micro pump 100 as the supply source 11 and includes only the mask 13 as an inspiratory pathway. In other words, the micro pump 100 is directly connected to the mask 13. The micro pump proposed in Patent Literature WO 2008/069266 is used as the micro pump 100. The micro pump 100 includes a primary blower chamber 101, and a secondary blower chamber 102 formed outside the primary blower chamber 101 as shown in Fig. 23(A).

The primary blower chamber 101 includes a piezoelectric element 103 to serve as a vibrating source, a diaphragm 104 to which the piezoelectric element 103 is fixed; and a vibration frame 105 that forms a space together with the diaphragm 104. The vibration frame 105 has an opening 106 for moving a fluid between the inside and the outside of the primary blower chamber 101. The secondary blower chamber 102 has an inlet 107 on the diaphragm 104 side and an outlet 108 facing the opening 106.

In the above micro pump 100, when the piezoelectric element 103 causes the diaphragm 104 to resonate, a fluid moves between the primary blower chamber 101 and the secondary blower chamber 102. The fluid resistance resulting from such fluid movement causes the vibration frame 105 to resonate. The resonance of the diaphragm 104 and the vibration frame 105 causes the fluid to be drawn in from the inlet 107 and causes the fluid to be discharged from the outlet 108.

The micro pump 100 is suitable for use as a blower for conveying a gas and capable of conveyance without the use of a check valve. While the micro pump 100 is extremely small with a box shape having an outer dimension of about 20 mm x 20 mm x 2 mm, the micro pump 100 can convey air of up to about 1 L/min (under the static pressure of 0 Pa) when the input sine wave has 15 Vpp (volt peak to peak) at 26 kHz. Such a micro pump 100 can also obtain a static pressure of up to about 2 kPa (the flow rate is 0 L/min).

However, since a fluid is conveyed by the vibration of the diaphragm 104 caused by the piezoelectric element 103, the micro pump 100 has naturally a limitation in the volume of a conveyable fluid. Thus, this static pressure-flow rate characteristic also exhibits a straight line as shown in Fig. 23(B). More specifically, in order to obtain a static pressure of about 1 kPa, for example, the flow rate is 0.5 L/min.

When the Vpp value of the input sine wave is changed to 10 or 20, the amplitude of the piezoelectric element 103 also changes, thus causing the flow rate and the pressure to change. In other words, when the Vpp value of the input sine wave is changed smoothly, the flow rate and the pressure can be changed smoothly. Alternatively, a change in the frequency of the input sine wave can cause the flow rate and the pressure to change. In other words, when the frequency of the input sine wave is changed smoothly, the flow rate and the pressure can be changed smoothly. Note however that the flow rate and the pressure have upper limits according to the capacity of the piezoelectric element 103 and the strength or durability of the material. The micro pump 100 is typically used at the rated Vpp and frequency.

Although the monomorph (unimorph) structure in which the single piezoelectric element 103 is attached to the diaphragm 104 has been discussed here, it is apparent that a bimorph structure in which two piezoelectric elements are attached to each other to increase the amount of vibration may be employed.

Note that the respiratory assistance device of the present invention is not limited to the above-described embodiments. It will be appreciated that various modifications are possible without departing from the scope of the present invention. Moreover, the components of the above-described embodiment may be applied to other embodiments if applicable.

Note that the above-described opening and closing device can be applied not only to open and close a hole through which expiratory air passes but also to open and close a hole through which a fluid (a gas or a liquid) passes as well as to open and close a hole through which a solid passes.

## Claims

1. An opening and closing device comprising:
a separating member (13) having a separating surface in which a flow hole (13a) is opened;
an opening and closing mechanism (15) for opening and closing the flow hole (13a), wherein
the opening and closing mechanism (15) is movable along the separating surface between a first position and a second position each having a different aperture area of the flow hole (13a), and
when a direction perpendicular to a moving direction (D_{M1}, D_{M2}) of the opening and closing mechanism (15) is defined as a width direction (W), an aperture length (L_{W}) of the flow hole (13a) in the width direction increases or decreases from the first position toward the second position,
**characterised in that** the opening and closing device comprises a piezoelectric element (15a) for holding the opening and closing mechanism (15) and moving the opening and closing mechanism (15) by deformation thereof.

2. The opening and closing device according to claim 1, wherein
an aperture ratio of the flow hole (13a) when the opening and closing mechanism (15) is located in the first position is smaller than that when the opening and closing mechanism (15) is located in the second position, and
a rate of change in an aperture length of the flow hole (13a) in the moving direction of the opening and closing mechanism (15) is smaller at the first position than at the second position.

3. The opening and closing device according to claim 1 or 2, wherein a plurality of the flow holes (13a) are provided in the separating member (13).

4. The opening and closing device according to claim 3, wherein the opening and closing mechanism (15) closes the plurality of the flow holes (13a) simultaneously.

5. The opening and closing device according to any one of claims 1 to 4, wherein
the opening and closing mechanism (15) is movable among the first position, the second position, and a third position,
the first position is disposed between the second position and the third position,
the flow hole (13a) is closed at the first position, and
the flow hole (13a) is opened at the second position and the third position.

6. The opening and closing device according to claim 5, wherein a variation profile of the aperture length of the flow hole (13a) from the first position toward the third position is different from a variation profile of the aperture length of the flow hole (13a) from the first position toward the second position.

7. The opening and closing device according to claim 5, wherein a variation profile of the aperture length of the flow hole (13a) from the first position toward the third position is the same as a variation profile of the aperture length of the flow hole (13a) from the first position toward the second position.

8. The opening and closing device according to any one of claims 1 to 7, further comprising:
a controller for controlling the deformation of the piezoelectric element (15a).

9. The opening and closing device according to any one of claims 1 to 8, further comprising:
a controller for controlling deformation of the piezoelectric element (15a), and wherein
part of the piezoelectric element (15a) serves as the opening and closing mechanism (15).

10. The opening and closing device according to claim 8 or 9, wherein a travel amount of the opening and closing mechanism (15) corresponds to a level of a signal input from the controller to the piezoelectric element (15a).

11. The opening and closing device according to any one of claims 1 to 10, further comprising an aperture area adjusting member (53) movable along the separating surface, and wherein
a gap (G) through which part of the flow hole (13a) is exposed is formed between the aperture area adjusting member (53) and the opening and closing mechanism (15).

12. The opening and closing device according to claim 11, wherein the aperture area adjusting member (53) is connected to the opening and closing mechanism (15).

13. A respiratory assistance device comprising the opening and closing device according to any one of claims 1 to 12, wherein
the separating member (13) is formed by a mask for covering a nose or a mouth, and a communicating pipe communicated with a space formed inside the mask in a worn state.

14. The respiratory assistance device according to claim 13, wherein the flow hole (13a) is formed in the mask.

15. The respiratory assistance device according to claim 14, wherein the flow hole (13a) is formed in the communicating pipe.

16. The respiratory assistance device according to any one of claims 13 to 15, wherein the flow hole (13a) forms an expiratory pathway through which expiratory air exhaled from the nose or the mouth passes.

## Patentansprüche

1. Öffnungs- und Schließvorrichtung mit:
einem Separationselement (13) mit einer Separierfläche, in der ein Strömungsloch (13a) offen ist;
einem Öffnungs- und Schließmechanismus (15) zum Öffnen und Schließen des Strömungsloches (13a), wobei
der Öffnungs- und Schließmechanismus (15) entlang der Separierfläche zwischen einer ersten Position und einer zweiten Position bewegbar ist, die jeweils eine unterschiedliche Öffnungsfläche des Strömungsloches (13a) haben, und
wenn eine Richtung, die senkrecht zu einer Bewegungsrichtung (D_{M1}, D_{M2}) des Öffnungs- und Schließmechanismus (15) ist, als eine Breitenrichtung (W) definiert ist, eine Öffnungslänge (L_{W}) des Strömungsloches (13a) in der Breitenrichtung von der ersten Position zu der zweiten Position zunimmt oder abnimmt,
**dadurch gekennzeichnet, dass**
die Öffnungs- und Schließvorrichtung ein piezoelektrisches Element (15a) aufweist zum Halten des Öffnungs- und Schließmechanismus (15) und Bewegen des Öffnungs- und Schließmechanismus (15) durch seine Verformung.

2. Öffnungs- und Schließvorrichtung gemäß Anspruch 1, wobei
ein Öffnungsverhältnis des Strömungsloches (13a), wenn der Öffnungs- und Schließmechanismus (15) sich in der ersten Position befindet, geringer ist als dann, wenn der Öffnungs- und Schließmechanismus (15) sich in der zweiten Position befindet, und
eine Änderungsrate einer Öffnungslänge des Strömungsloches (13a) in der Bewegungsrichtung des Öffnungs- und Schließmechanismus (15) bei der ersten Position geringer ist als bei der zweiten Position.

3. Öffnungs- und Schließvorrichtung gemäß Anspruch 1 oder 2, wobei eine Vielzahl an Strömungslöchern (13a) in dem Separierelement (13) vorgesehen sind.

4. Öffnungs- und Schließvorrichtung gemäß Anspruch 3, wobei der Öffnungs- und Schließmechanismus (15) die Vielzahl der Strömungslöcher (13a) gleichzeitig schließt.

5. Öffnungs- und Schließvorrichtung gemäß einem der Ansprüche 1 bis 4, wobei
der Öffnungs- und Schließmechanismus (15) zwischen der ersten Position, der zweiten Position und einer dritten Position bewegbar ist,
wobei die erste Position zwischen der zweiten Position und der dritten Position angeordnet ist,
das Strömungsloch (13a) an der ersten Position geschlossen ist, und das Strömungsloch (13a) an der zweiten Position und der dritten Position offen ist.

6. Öffnungs- und Schließvorrichtung gemäß Anspruch 5, wobei ein Variationsprofil der Öffnungslänge des Strömungsloches (13a) von der ersten Position zu der dritten Position anders ist als ein Variationsprofil der Öffnungslänge des Strömungsloches (13a) von der ersten Position zu der zweiten Position.

7. Öffnungs- und Schließvorrichtung gemäß Anspruch 5, wobei ein Variationsprofil der Öffnungslänge des Strömungsloches (13a) von der ersten Position zu der dritten Position das gleiche ist wie ein Variationsprofil der Öffnungslänge des Strömungsloches (13a) von der ersten Position zu der zweiten Position.

8. Öffnungs- und Schließvorrichtung gemäß einem der Ansprüche 1 bis 7, die des Weiteren Folgendes aufweist:
eine Steuereinrichtung zum Steuern der Verformung des piezoelektrischen Elementes (15a).

9. Öffnungs- und Schließvorrichtung gemäß einem der Ansprüche 1 bis 8, die des Weiteren Folgendes aufweist:
eine Steuereinrichtung zum Steuern einer Verformung des piezoelektrischen Elementes (15a), und wobei
ein Teil des piezoelektrischen Elementes (15a) als der Öffnungs- und Schließmechanismus (15) dient.

10. Öffnungs- und Schließvorrichtung gemäß Anspruch 8 oder 9, wobei ein Bewegungsbetrag des Öffnungs- und Schließmechanismus (15) einer Höhe eines Signals entspricht, das von der Steuereinrichtung zu dem piezoelektrischen Element (15a) eingegeben wird.

11. Öffnungs- und Schließvorrichtung gemäß einem der Ansprüche 1 bis 10, die des Weiteren ein Öffnungsbereicheinstellelement (53) aufweist, das entlang der Separierfläche bewegbar ist, und wobei
ein Zwischenraum (G), durch den ein Teil des Strömungsloches (13a) freigelegt ist, zwischen dem Öffnungsbereicheinstellelement (53) und dem Öffnungs- und Schließmechanismus (15) ausgebildet ist.

12. Öffnungs- und Schließvorrichtung gemäß Anspruch 11, wobei das Öffnungsbereicheinstellelement (53) mit dem Öffnungs- und Schließmechanismus (15) verbunden ist.

13. Beatmungsunterstützungsvorrichtung der Öffnungs- und Schließvorrichtung gemäß einem der Ansprüche 1 bis 12, wobei
das Separierelement (13) durch eine Maske zum Ausbilden einer Nase oder eines Mundes und ein Kommunikationsrohr ausgebildet ist, das mit einem Raum in Kommunikation steht, der im Inneren der Maske in einem aufgesetzten Zustand ausgebildet ist.

14. Beatmungsunterstützungsvorrichtung gemäß Anspruch 13, wobei das Strömungsloch (13a) in der Maske ausgebildet ist.

15. Beatmungsunterstützungsvorrichtung gemäß Anspruch 14, wobei das Strömungsloch (13a) in dem Kommunikationsrohr ausgebildet ist.

16. Beatmungsunterstützungsvorrichtung gemäß einem der Ansprüche 13 bis 15, wobei das Strömungsloch (13a) einen Ausatmungspfad ausbildet, durch den die von der Nase oder dem Mund ausgeatmete Ausatemluft tritt.

## Revendications

1. Dispositif d'ouverture et de fermeture comprenant :
un élément de séparation (13) ayant une surface de séparation dans laquelle un trou d'écoulement (13a) est ouvert ;
un mécanisme d'ouverture et de fermeture (15) pour ouvrir et fermer le trou d'écoulement (13a),
dans lequel
le mécanisme d'ouverture et de fermeture (15) est mobile le long de la surface de séparation entre une première position et une deuxième position, chacune ayant une zone d'ouverture différente du trou d'écoulement (13a), et
lorsqu'une direction perpendiculaire à une direction de déplacement (D_{M1}, D_{M2}) du mécanisme d'ouverture et de fermeture (15) est définie comme étant une direction de largeur (W), une longueur d'ouverture (L_{W}) du trou d'écoulement (13a) dans la direction de largeur, augmente ou diminue de la première position vers la deuxième position,
**caractérisé en ce que** le dispositif d'ouverture et de fermeture comprend un élément piézoélectrique (15a) pour maintenir le mécanisme d'ouverture et de fermeture (15) et déplacer le mécanisme d'ouverture et de fermeture (15) par sa déformation.

2. Dispositif d'ouverture et de fermeture selon la revendication 1, dans lequel :
un rapport d'ouverture du trou d'écoulement (13a) lorsque le mécanisme d'ouverture et de fermeture (15) est positionné dans la première position, est inférieur au rapport d'ouverture lorsque le mécanisme d'ouverture et de fermeture (15) est positionné dans la deuxième position, et
un taux de changement dans une longueur d'ouverture du trou d'écoulement (13a) dans la direction de déplacement du mécanisme d'ouverture et de fermeture (15) est moins important dans la première position que dans la deuxième position.

3. Dispositif d'ouverture et de fermeture selon la revendication 1 ou 2, dans lequel une pluralité de trous d'écoulement (13a) sont prévus dans l'élément de séparation (13).

4. Dispositif d'ouverture et de fermeture selon la revendication 3, dans lequel le mécanisme d'ouverture et de fermeture (15) ferme la pluralité de trous d'écoulement (13a) simultanément.

5. Dispositif d'ouverture et de fermeture selon l'une quelconque des revendications 1 à 4, dans lequel :
le mécanisme d'ouverture et de fermeture (15) est mobile parmi la première position, la deuxième position et une troisième position,
la première position est disposée entre la deuxième position et la troisième position,
le trou d'écoulement (13a) est fermé dans la première position, et
le trou d'écoulement (13a) est ouvert dans la deuxième position et la troisième position.

6. Dispositif d'ouverture et de fermeture selon la revendication 5, dans lequel un profil de variation de la longueur d'ouverture du trou d'écoulement (13a) de la première position vers la troisième position est différent d'un profil de variation de la longueur d'ouverture du trou d'écoulement (13a) de la première position vers la deuxième position.

7. Dispositif d'ouverture et de fermeture selon la revendication 5, dans lequel un profil de variation de la longueur d'ouverture du trou d'écoulement (13a) de la première position vers la troisième position est le même qu'un profil de variation de la longueur d'ouverture du trou d'écoulement (13a) de la première position vers la deuxième position.

8. Dispositif d'ouverture et de fermeture selon l'une quelconque des revendications 1 à 7, comprenant en outre :
un organe de commande pour commander la déformation de l'élément piézoélectrique (15a).

9. Dispositif d'ouverture et de fermeture selon l'une quelconque des revendications 1 à 8, comprenant en outre :
un organe de commande pour commander la déformation de l'élément piézoélectrique (15a), et dans lequel :
une partie de l'élément piézoélectrique (15a) sert de mécanisme d'ouverture et de fermeture (15).

10. Dispositif d'ouverture et de fermeture selon la revendication 8 ou 9, dans lequel une quantité de déplacement du mécanisme d'ouverture et de fermeture (15) correspond à un niveau d'une entrée de signal, de l'organe de commande à l'élément piézoélectrique (15a).

11. Dispositif d'ouverture et de fermeture selon l'une quelconque des revendications 1 à 10, comprenant en outre un élément d'ajustement de zone d'ouverture (53) mobile le long de la surface de séparation, et dans lequel :
un espace (G) à travers lequel une partie du trou d'écoulement (13a) est exposée, est formé entre l'élément d'ajustement de zone d'ouverture (53) et le mécanisme d'ouverture et de fermeture (15).

12. Dispositif d'ouverture et de fermeture selon la revendication 11, dans lequel l'élément d'ajustement de zone d'ouverture (53) est raccordé au mécanisme d'ouverture et de fermeture (15).

13. Dispositif d'assistance respiratoire comprenant le dispositif d'ouverture et de fermeture selon l'une quelconque des revendications 1 à 12, dans lequel :
l'élément de séparation (13) est formé par un masque pour recouvrir un nez ou une bouche, et un tuyau de communication qui communique avec un espace formé à l'intérieur du masque dans un état porté.

14. Dispositif d'assistance respiratoire selon la revendication 13, dans lequel le trou d'écoulement (13a) est formé dans le masque.

15. Dispositif d'assistance respiratoire selon la revendication 14, dans lequel le trou d'écoulement (13a) est formé dans le tuyau de communication.

16. Dispositif d'assistance respiratoire selon l'une quelconque des revendications 13 à 15, dans lequel le trou d'écoulement (13a) forme une voie de passage d'expiration à travers laquelle passe l'air d'expiration expiré par le nez ou la bouche.
